# EUROPEAN PATENT APPLICATION

(11) **EP 4 006 994 A1**
(43) Date of publication of application: **01.06.2022**
(21) Application number: 20306452.2
(22) Date of filing: 26.11.2020
(51) Int. Cl.: H01L 31/173, H01L 31/0352

(54) **OPTOELECTRONIC DEVICE**

(71) Applicant: STMICROELECTRONICS (GRENOBLE 2) SAS, 38000 Grenoble (FR)
(72) Inventor: STECKEL, Jonathan, 38700 CORENC (FR); ROCHEREAU, Krysten, 38580 ALLEVARD (FR)
(74) Representative: Cabinet Beaumont

(57) **Abstract**

The present disclosure relates to an optoelectronic device (10) comprising, on a same substrate (16), a light emitter (12) and a photodetector (14), at least one among the emitter and the photodetector being based on semiconductor nanoparticles.

## Description

### Technical field

The present disclosure relates generally to optoelectronic devices, and more particularly to devices comprising a light emitter and a light sensor.

### Background art

Numerous optoelectronic devices comprise a light emitter and a light photodetector, or photodetector. The emitter is configured to generate light when a specific voltage is applied to the emitter. The photodetector is configured to generate electron-hole pairs or photocurrent when receiving light, preferably the light emitted by the emitter.

### Summary of Invention

One embodiment addresses all or some of the drawbacks of known optoelectronic devices.

One embodiment provides an optoelectronic device comprising, on a same substrate, a light emitter and a photodetector, at least one among the emitter and the photodetector being based on semiconductor nanoparticles.

Another embodiment provides a method of manufacturing an optoelectronic device comprising the formation, on a same substrate, of a light emitter and of a photodetector, at least one among the emitter and the photodetector being based on semiconductor nanoparticles.

According to an embodiment, each photodetector or emitter based on semiconductor nanoparticles comprises a layer of semiconductor nanoparticles, a hole transport layer, an electron transport layer and electrodes.

According to an embodiment, the device comprises an emitter based on semiconductor nanoparticles and a semiconductor-based photodetector.

According to an embodiment, the photodetector comprises a photodiode.

According to an embodiment, the photodetector does not comprise semiconductor nanoparticles.

According to an embodiment, the light emitter and the photodetector are side by side on the substrate.

According to an embodiment, the light emitter and the photodetector are stacked.

According to an embodiment, both the light emitter and the photodetector are based on semiconductor nanoparticles, and are stacked.

According to an embodiment, both the light emitter and the photodetector are separated by an insulating layer.

According to an embodiment, the substrate is curved.

According to an embodiment, the core of the semiconductor particles is made of a material among the following or an alloy of materials among the following: CdSe, CdS, CdTe, CdSeS, CdTeSe, AgS, ZnO, ZnS, ZnSe, CuInS, CuInSe, CuInGaS, CuInGaSe, PbS, PbSe, PbSeS, PbTe, InAsSb, InAs, InSb, InGaAs, InP, InGaP, InAlP, InGaAlP, InZnS, InZnSe, InZnSeS, HgTe, HgSe, HgSeTe, Ge, Si, and the shell of the semiconductor particles is made of a material among the following or an alloy of materials among the following: CdSe, CdS, CdTe, CdSeS, CdTeSe, AgS, ZnO, ZnS, ZnSe, CuInS, CuInSe, CuInGaS, CuInGaSe, PbS, PbSe, PbSeS, PbTe, InAsSb, InAs, InSb, InGaAs, InP, InGaP, InAlP, InGaAlP, InZnS, InZnSe, InZnSeS, HgTe, HgSe, HgSeTe, Ge, Si.

Another embodiment provides a spectrometer comprising a device as described previously.

Another embodiment provides an image sensor comprising a device as described previously.

Another embodiment provides a hyperspectral image sensor comprising a device as described previously.

Another embodiment provides a multispectral image sensor comprising a device as described previously.

Another embodiment provides a proximity sensor comprising a device as described previously.

Another embodiment provides a light communication device comprising a device as described previously.

Another embodiment provides a time of flight sensor comprising a device as described previously.

Another embodiment provides a finger print sensor comprising a device as described previously.

Another embodiment provides a face-ID sensor comprising a device as described previously.

Another embodiment provides a medical, health, or wellness sensor comprising a device as described previously.

### Brief description of drawings

The foregoing features and advantages, as well as others, will be described in detail in the following description of specific embodiments given by way of illustration and not limitation with reference to the accompanying drawings, in which:
Figure 1 illustrates an embodiment of an optoelectronic device;
Figure 2 illustrates different light wavelengths and spectra potentially emitted by an optoelectronic device;
Figure 3 illustrates another embodiment of an optoelectronic device;
Figure 4 illustrates another embodiment of an optoelectronic device;
Figure 5 illustrates another embodiment of an optoelectronic device; and
Figure 6 illustrates another embodiment of an optoelectronic device.

### Description of embodiments

Like features have been designated by like references in the various figures. In particular, the structural and/or functional features that are common among the various embodiments may have the same references and may dispose identical structural, dimensional and material properties.

For the sake of clarity, only the operations and elements that are useful for an understanding of the embodiments described herein have been illustrated and described in detail.

Unless indicated otherwise, when reference is made to two elements connected together, this signifies a direct connection without any intermediate elements other than conductors, and when reference is made to two elements coupled together, this signifies that these two elements can be connected or they can be coupled via one or more other elements.

In the following disclosure, unless indicated otherwise, when reference is made to absolute positional qualifiers, such as the terms "front", "back", "top", "bottom", "left", "right", etc., or to relative positional qualifiers, such as the terms "above", "below", "higher", "lower", etc., or to qualifiers of orientation, such as "horizontal", "vertical", etc., reference is made to the orientation shown in the figures.

Unless specified otherwise, the expressions "around", "approximately", "substantially" and "in the order of" signify within 10 %, and preferably within 5 %.

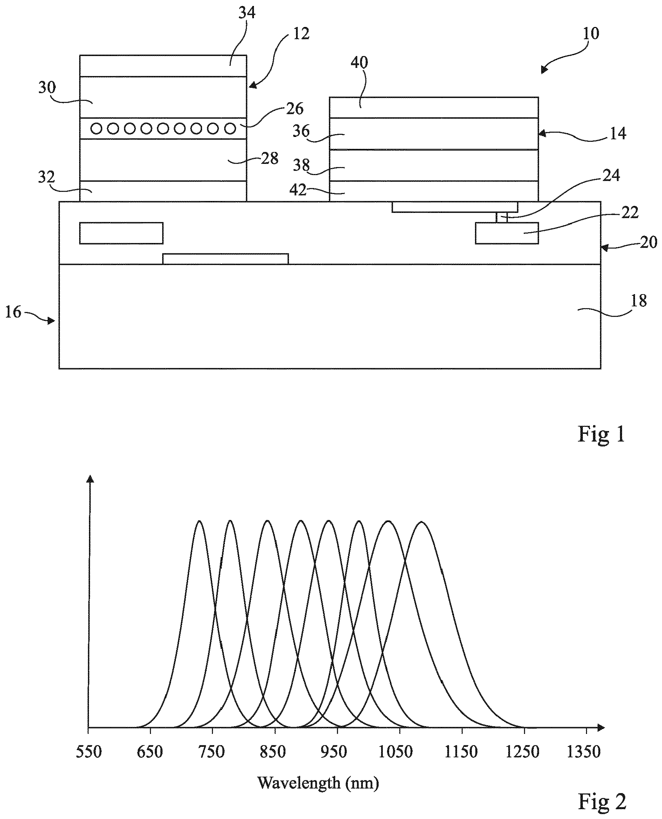

Figure 1 illustrates an embodiment of an optoelectronic device 10.

The device 10 comprises a light emitter 12 and a photodetector 14, configured to receive at least part of the light emitted by the emitter 12 preferably after reflection of the light on a scene.

The device 10 is for example a time of flight (ToF) sensor, in other words, the device for example aims at measuring the distance by time of flight (ToF). The device is for example a proximity sensor. The device 10 is for example a spectrometer. The device 10 is for example configured to be used in LiFi (Light Fidelity) or, in other words, used to allow wireless transmission of information through light. For example, device 10 is configured to be used in an imager, or image sensor, in order to generate images representing the scene. For example, the device is a hyperspectral image sensor or a multispectral image sensor. The device is for example a fingerprint sensor. The device is for example a face-ID sensor, in other words a device configured to identify a face. For example, the device is a medical, health, or wellness sensor.

The device 10 comprises a support 16. The emitter 12 and the photodetector 14 are located side by side on the same support 16. The support 16 comprises a substrate 18. Therefore, the emitter 12 and the photodetector 14 are located on the same substrate. The substrate 18 is for example a semiconductor layer, for example in silicon. For example, electronic components are located in and on the substrate 18. For example, transistors (not represented), for example MOS (Metal Oxide Semiconductor) transistors, for example CMOS (Complementary Metal Oxide Semiconductor) transistors, can be formed in and on the substrate 18. Alternatively, the substrate can be of another material, for example of glass or of plastic.

The support 16 preferably comprises an interconnection network 20 located on substrate 18, preferably semiconductor substrate 18. The interconnection network 20 comprises a stack of insulating layers surrounding several levels of conductive pads 22, the pads 22 being interconnected by conductive vias 24. The interconnection network allows interconnection of the components formed in and on the substrate between themselves and with the emitter and the photodetector.

The emitter 12 is a light emitter based on semiconductor nanoparticles, often called quantum dots. More particularly, the emitter 12 comprises a layer, or multiple layers, 26 of semiconductor nanoparticles.

A semiconductor nanoparticle is a nanoscopic material structure which, given an electrical excitation corresponding to its band gap, emits photons due to the production and subsequent recombination of election-hole pairs inside the semiconductor nanoparticles. Similarly, electron-hole pairs can be produced given the incidence of photons onto the nanoscopic material structure. In this manner, it is possible to create emitting elements such as LEDs (Light Emitting Diode) for example, and detecting elements such as photodetectors for example, on the basis of semiconductor nanoparticles.

A semiconductor nanoparticle comprises a semiconductor core. The dimensions or size, composition, and shape of the core determine the wavelength of the light emitted by the emitter, if the semiconductor nanoparticle is part of a light emitter, or the wavelength of the light absorbed generating the election-hole pairs in a photodetector. A semiconductor nanoparticle can also comprise a shell, preferably in a semiconductor material, surrounding the core in order to protect and passivate the core. A semiconductor nanoparticle further comprises ligands, organic aliphatics, organometallic, or inorganic molecules that extend from the shell and passivate, protect, and functionalise the semiconductor surface.

Figure 2 illustrates different light wavelengths, potentially emitted by an optoelectronic device, for example by the emitter 12 of Figure 1. More precisely, Figure 2 illustrates the quantity of emission (Y axis) as a function of the wavelength (X axis, in nm) for several types and or sizes of semiconductor nanoparticle cores, for example for cores and shells in different materials.

Each curve, corresponding to the quantity of emission of one type of semiconductor nanoparticle, has substantially the form of a Gaussian curve. The maximal value of each curve corresponds to the central wavelength of the light emitted by the emitter.

As shown in Figure 2, the range of wavelengths that can be emitted by emitter 12 is quite large. For example, the wavelengths that can be emitted by emitter 12 are from 300 nm and above, and for example in the range from 300 nm to 3000 nm. It is therefore possible to tune, or adjust, emitter 12 by choosing the features of the semiconductor nanoparticles, in order to obtain a light emitter configured to emit a chosen wavelength.

With reference again to Figure 1, the semiconductor nanoparticles of the layer 26 can be among several types of semiconductor nanoparticles. For example, the semiconductor nanoparticles can be quantum dots, in other words a type of semiconductor nanoparticles with a core substantially spherical. The semiconductor nanoparticles can also be quantum wires, or quantum rods, in other words a type of semiconductor nanoparticles with a core having an extended form in one direction, for example substantially the form of a cylinder. The semiconductor nanoparticles can also be quantum wells, in other words a type of semiconductor nanoparticles with a core having substantially the form of a layer, in other words the form of a parallelepiped.

The core is, for example, made of a material among the following or an alloy of materials among the following: CdSe, CdS, CdTe, CdSeS, CdTeSe, AgS, ZnO, ZnS, ZnSe, CuInS, CuInSe, CuInGaS, CuInGaSe, PbS, PbSe, PbSeS, PbTe, InAsSb, InAs, InSb, InGaAs, InP, InGaP, InAlP, InGaAlP, InZnS, InZnSe, InZnSeS, HgTe, HgSe, HgSeTe, Ge, Si. The shell is, for example, made of a material among the following or an alloy of materials among the following: CdSe, CdS, CdTe, CdSeS, CdTeSe, AgS, ZnO, ZnS, ZnSe, CuInS, CuInSe, CuInGaS, CuInGaSe, PbS, PbSe, PbSeS, PbTe, InAsSb, InAs, InSb, InGaAs, InP, InGaP, InAlP, InGaAlP, InZnS, InZnSe, InZnSeS, HgTe, HgSe, HgSeTe, Ge, Si. The choice of the materials depends on the desired wavelength, as described in relation with Figure 2.

Preferably, the dimensions of the core are lower than 20 nm, for example in the range from 2 to 15 nm. In particular, in the case of quantum dots, the diameter of each quantum dot is preferably in the range from 2 to 15 nm.

The layer 26 is located between an electron transport layer 28, constituting the cathode, and a hole transport layer 30, constituting the anode. In the example of Figure 1, the hole transport layer 30 is located above the layer 26 and the electron transport layer 28 is located below the layer 26. The electron transport layer 28 is therefore in contact with the face of the layer 26 that is closest to the substrate 18 and the hole transport layer 30 is therefore in contact with the face of the layer 26 that is farthest from the substrate 18. The electron transport layer 28 is in contact with an electrode layer, or electrode, 32 and the electron transport layer 28 is in contact with an electrode layer, or electrode, 34.

The electrode layers 32 and 34 are made of conductive material, for example in metal or in indium tin oxide. The electrodes 32 and 34 allow a voltage to be applied between the anode and the cathode of the emitter.

The closest electrode to the substrate, in this example the electrode 32, is for example located on, and therefore above, the interconnection network 20. Alternatively, the closest electrode to the substrate can be a pad 22 of the network 20.

The emitter 12 of Figure 1 for example comprises a stack of layers comprising, from the support 16, the electrode 32, the electron transport layer 28, the semiconductor nanoparticles layer 26, the hole transport layer 30 and the electrode 34.

Several layers of the emitter are preferably at least partially transparent to the wavelengths of the light emitted by the emitter 12. More particularly, the layers located between the layer 26 and the scene towards which the light is emitted are at least partially transparent to the wavelengths of the light emitted by the emitter. In the example of Figure 1, the light is emitted on the side opposite to the substrate, in other words through the hole transport layer 30 and the electrode 34. The hole transport layer 30 and the electrode 34 are therefore preferably at least partially transparent.

According to another embodiment, the light can be emitted through the substrate 18. The layers located between the layer 26 and the support 16, in this example the electron transport layer 28 and the electrode 32, the substrate 18 and the isolating layers of the network 20 are at least partially transparent to the wavelengths of the light emitted by the emitter. Preferably, no component or pad is located on the path of the light emitted by the emitter 12, in other words no component or pad is located in vertical alignment with the layer 26.

The feature of being at least partially transparent is for example an intrinsic feature of the materials or a consequence of the thickness of the layers. Preferably, at least one of the layers of the emitter, preferably all the layers of the emitter, are thin layers, for example having a thickness lower than 200 nm. Therefore, those layers are partially transparent to the wavelength emitted and received by the emitter and the photodetector.

The photodetector 14 is configured to receive at least part of the light emitted by the emitter, preferably after reflection on a scene. The photodetector is preferably a semiconductor-based photodetector. Preferably, a photodetector comprising a photodiode. Therefore, the photodetector preferably comprises semiconductor layers forming a PN junction or a PIN junction. The junction can be for example a vertical junction, or a horizontal junction. The photodetector 14 does not, preferably, comprise a layer of semiconductor nanoparticles.

In Figure 1, the photodetector 14 comprises a photodiode with a horizontal PN junction. The photodetector 14 comprises an n-doped semiconductor layer 36 and a p-doped semiconductor layer 38, forming the PN junction. In the example of Figure 1, the layer 36 is in contact with, and located above, layer 38. The photodetector also comprises, in the example of Figure 1, an electrode 40 in contact with layer 36 and an electrode 42 in contact with layer 38. The photodetector 14 therefore comprises a stack of layers comprising, from the support 20, the electrode 42, the semiconductor layer 38, the semiconductor layer 36, and the electrode 40.

Preferably, as in the emitter, at least some of the layers of the photodetector 14 are at least partially transparent to the wavelengths of the light the photodetector is configured to be sensitive to, in other words the wavelength which is configured to generate hole-electron pairs in the photodetector. For example, the electrode 40 is at least partially transparent to the wavelengths of the light the photodetector is configured to be sensitive to.

In the example of Figure 1, the emitter and the photodetector are separated by air, in other words by empty space. Alternatively, the emitter and the photodetector can be separated by one or several materials. Preferably, the emitter and the photodetector are separated by at least an insulating material, preferably by a material opaque to the wavelengths emitted by the emitter and the wavelengths which are configured to generate hole-electron pairs in the photodetector.

The features of the semiconductor particles of the emitter are chosen so that the emitter emits light having wavelengths in a first range, for example centered on a first wavelength, and the features of the photodetector are chosen so that the photodetector generate electron-hole pairs when receiving light having wavelengths in a second range, for example centered on a second wavelength.

The first and second wavelength can for example be substantially identical. According to another embodiment, the first and second wavelength can be different, in order to receive a maximum light from the emitter. Indeed, in some applications, the light emitted by the emitter can change wavelength when interacting with the scene. Therefore, the light emitted by the emitter is not the same wavelength as the light reflected towards the photodetector.

While Figure 1 represents some details of the emitter and the photodetector, they can, according to another embodiment, be replaced by other types of semiconductor nanoparticle-based emitters and photodetectors not comprising semiconductor nanoparticles.

The formation of the device comprises the formation, on the same substrate 18, of the emitter and of the photodetector. For example, the emitter and the photodetector are formed sequentially. In other words, the emitter or the photodetector is formed first then the other is formed, for example while the region of the element not being formed is protected by a mask. In the case the emitter and the photodetector comprise common layers, for example among the layers the closest to the substrate, those layers can be formed simultaneously. An example of a method of manufacturing can comprise, preferably in succession:
the formation of the substrate 18;
the formation, if relevant, of the components of the substrate 18;
the formation of the interconnection network 20;
the formation of a mask protecting the region in which the photodetector is to be formed;
the formation of the emitter, comprising the formation of the electrode 32, the formation of the electron transport layer 28, the formation of the semiconductor nanoparticles layer 26, the formation of the hole transport layer 30 and the formation of the electrode 34;
the removal of the mask protecting the localization of the photodetector;
the formation of a mask protecting the emitter; and
the formation of the photodetector, comprising the formation of the electrode 42, the formation of the p-doped semiconductor layer 38, the formation of the n-doped semiconductor layer, and the formation of the electrode 40.

Alternatively, the photodetector can be formed before the emitter.

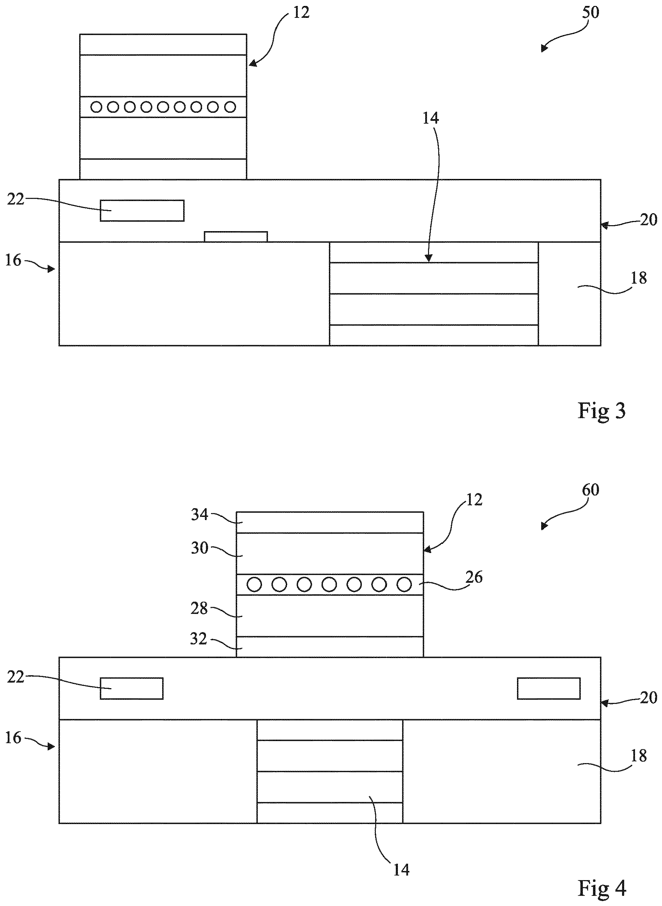

Figure 3 illustrates another embodiment of an optoelectronic device 50.

The device 50 comprises, like the device 10 of Figure 1, the light emitter 12 and the photodetector 14, configured to receive at least part of the light emitted by the emitter 12. Like the device 10, the device 50 comprises the support 16, and therefore comprises the semiconductor substrate 18 and the interconnection network 20.

The device 50 differs from the device 10 in that the photodetector 14 is not located on the support, and therefore on the substrate, but in the substrate. For example, in the case in which the photodetector comprises a photodiode, semiconductor regions are formed in the substrate 18 in order to form a PN junction or a PIN junction.

Preferably, the layers covering the photodetector, in other words the layers located between the photodetector and the scene from which the light emitted by the emitter are reflected, are transparent to the wavelengths which are configured to generate hole-electron pairs in the photodetector.

Preferably, the emitter 12 is offset to the side of the photodetector 14. In other words, the emitter is located in vertical alignment with a region of the substrate 18 outside of the photodetector 14. In other words, the emitter is not located in vertical alignment with the photodetector. In other words, the emitter does not cover, even partially, the photodetector.

An example of a method of manufacturing this embodiment differs from the method of manufacturing the embodiment of Figure 1 in that the formation of the photodetector is part of the formation of the components of the substrate, and is therefore done before the formation of the emitter.

Figure 4 illustrates another embodiment of an optoelectronic device 60. Figure 4 comprises the same elements as Figure 3. Only the differences between the device 50 and the device 60 will be highlighted.

The device 60 differs from the device 50 in that the emitter 12 is located in vertical alignment with the photodetector 14. In other words, the emitter at least partially covers the photodetector, preferably entirely covers the photodetector. In particular, the active zone of the emitter, in other words the region of the emitter from which the light is emitted, for example the layer 26 of semiconductor particles, is located in vertical alignment with at least part of the active zone of the photodetector, in other words the region of the photodetector destined to receive the light emitted by the emitter.

The light emitted by the emitter is sent in the direction of a scene. The layers of the device 60 located between the active zone of the emitter are preferably at least partially transparent to the wavelength of the light emitted by the emitter. In the example of Figure 4, the scene is located on the side of the emitter opposed to the photodetector. Therefore, the layers of the device 60 located between the active zone and the scene are the hole transport layer 30 and the electrode 34. Some of the light emitted is for example reflected on the scene and reaches the photodetector, for example through the emitter. Preferably, all the layers of the emitter 12 located between the scene and the photodetector are at least partially transparent to the wavelength of the light generating the electron-hole pairs in the photodetector. In the example of Figure 4, the layers located between the photodetector and the scene comprise all the layers of the emitter, in other words the electrodes 32 and 34, the electron transport layer 28, the hole transport layer 30 and the semiconductor particles layer 26, as well as the interconnection network 20. Preferably, no pad 22 is located in the interconnection network between the photodetector and the scene.

The method of manufacturing this embodiment is similar to the method of manufacturing the embodiment of Figure 3, but differs in that the emitter is formed in vertical alignment with the photodetector.

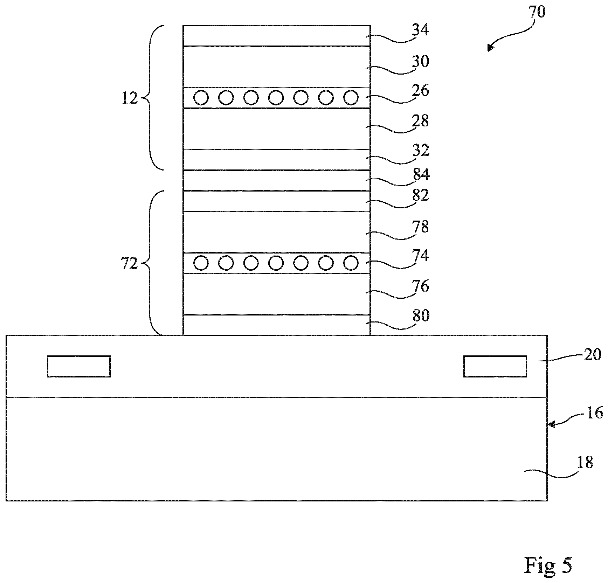

Figure 5 illustrates another embodiment of an optoelectronic device 70. The device 70 comprises, on the support 16, a stack of the emitter 12 and a photodetector 72 based on semiconductor particles.

The device 70 comprises the support 16, as described previously, and in particular comprises the interconnection network 20 and the semiconductor substrate 18. The device 70 comprises the emitter 12, in other words comprises the electrodes 32 and 34, the electron transport layer 28, the hole transport layer 30 and the semiconductor particles layer 26.

The device 70 further comprises the photodetector 72 based on semiconductor particles. The photodetector 72 comprises a layer 74 comprising semiconductor nanoparticles as described previously. The layer 74 is configured to generate electron-hole pairs when receiving light at wavelength within the working range of the photodetector.

The different types of semiconductor nanoparticles have already been described in relation with Figure 1. The semiconductor particles of layer 74 are of the same type (quantum dots, quantum wire or rod, quantum well) as, or are of a different type to, the layer 26. Similarly, the material of the particles of layer 74 is for example among the same list of materials described in relation with Figure 1. The particles of layer 26 and the particles of layer 74 are for example of different materials or of the same material. Similarly, the particles of layer 26 and the particles of layer 74 can have substantially the same dimensions or can be of different dimensions. Preferably, the particles of layer 26 and the particles of layer 74 have different dimensions and/or are of different type and/or are of different materials to each other.

More generally, the features of the semiconductor particles of the emitter are chosen so that the emitter emits light having wavelengths in a first range, for example centered on a first wavelength, and the features of the semiconductor particles of the photodetector are chosen so that the photodetector generate electron-hole pairs when receiving light having wavelengths in a second range, for example centered on a second wavelength.

The first and second wavelengths can for example be substantially identical. According to another embodiment, the first and second wavelengths can be different, as described previously.

The layer 74 is located between an electron transport layer 76, constituting the cathode, and a hole transport layer 78, constituting the anode. In the example of Figure 5, the hole transport layer 78 is located above the layer 74 and the electron transport layer 76 is located below the layer 74. The electron transport layer 76 is therefore in contact with the face of the layer 74 closest to the substrate 18 and the hole transport layer 78 is therefore in contact with the face of the layer 26 farthest from the substrate 18. The electron transport layer 76 is in contact with an electrode layer, or electrode, 80 and the electron transport layer 76 is in contact with an electrode layer, or electrode, 82.

The electrode layers 80 and 82 are made of conductive material, for example of metal. The electrodes 80 and 82 allow a voltage to be applied between the anode and the cathode of the anode of the photodetector.

The electrode closest to the substrate, in this example the electrode 80, is, in the example of Figure 5, located on, and therefore above, the interconnection network 20. Alternatively, the electrode closest to the substrate can be a pad 22 of the network 20.

Therefore, the photodetector 72 comprises a stack of layers comprising, from the support 16, the electrode 80, the electron transport layer 76, the semiconductor nanoparticles layer 74, the hole transport layer 78 and the electrode 82.

The emitter and the photodetector are stacked one upon the other. In the example of Figure 5, the emitter is located on the photodetector. The photodetector is located on the support 16. Therefore, the photodetector 72 is located between the emitter 12 and the support 16.

Alternatively, the emitter can be located below the photodetector. The emitter is therefore located between the photodetector and the support.

As in the embodiment of Figure 4, at least some parts of the device of Figure 5 are preferably at least partially transparent to the wavelength of the light emitted by the emitter 12 and/or to the wavelength of the light received by the photodetector.

For example, the device 70 comprises an insulating layer 84 located between the emitter 12 and the photodetector 72. The insulating layer 84 is for example between the electrode 32 of the emitter and the electrode 82 of the photodetector. The insulating layer 84 is for example in contact with, on one side, the electrode 32 of the emitter and, on the other side, with the electrode 82 of the photodetector. The insulating layer 84 ensures the electrical insulation of the emitter and the photodetector. In particular, the layer 84 ensures that there is no electrical connection between the electrode 82 and the electrode 32. The emitter and the photodetector can therefore be controlled independently by applying different voltages between the electrodes 32 and 34 and between the electrodes 80 and 82.

Alternatively, the emitter and the photodetector can have a common electrode replacing the electrodes 82 and 32 and the insulating layer 84.

An advantage of the embodiment of Figure 5 is that the working wavelengths of the emitter and of the photodetector are tunable by choosing the features of the semiconductor particles.

Another advantage of the embodiment of Figure 5 is that the surface occupied by the device is lower than the surface occupied if the emitter and the photodetector were located side by side.

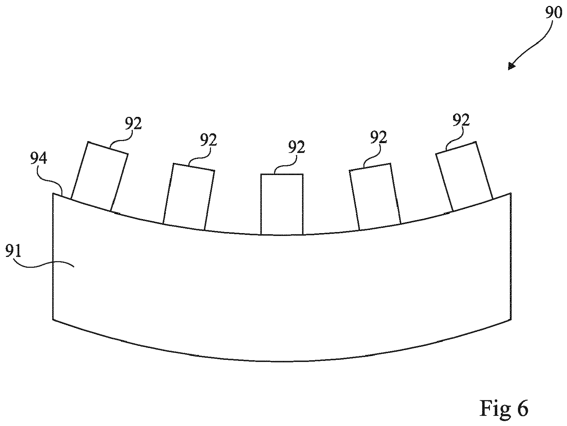

Figure 6 illustrates another embodiment of an optoelectronic device 90.

The device 90 comprises a curved substrate 91. The substrate is for example of a semiconductor material, of glass, of metal, or of a plastic material. Optoelectrical elements 92 are formed on a face 94 of the substrate.

By a curved substrate, we mean a substrate comprising at least one curved face, preferably a horizontal face, preferably the face on which components are formed. In other words, we mean a substrate comprising at least one face having edges, preferably all of its edges, on a different level from the level of the centre of the face. Preferably, both horizontal faces have edges on a different level with respect to the level of the centres of the faces.

Preferably, at least one horizontal face of the substrate has a radius of curvature of less than 1 cm, enabling curvature-tunable devices.

In the example of Figure 6, both horizontal faces of the substrate are curved. Each element 92 is for example a light emitter or a photodetector.

According to an embodiment, the device 90 comprises a single emitter and a plurality of photodetectors, both emitter and photodetectors being represented by an element 92. For example, the emitter is located at the center of the face 94. Preferably, the emitter is a semiconductor nanoparticles-based emitter, for example the emitter 12 described previously. The photodetectors of the device 90 are for example similar to the photodetector 14 described previously, in other words a photodetector not comprising semiconductor nanoparticles.

According to another embodiment, the device 90 comprises a plurality of emitters 12 and a plurality of photodetectors 14, each represented by an element 92.

According to another embodiment, each element 92 can represent a photodetector and an emitter. For example, each element represents the emitter 12 and the photodetector 14 or 72 of the embodiment of Figure 1, 3, 4 or 5.

It would be possible to form, separately, the emitter and the photodetector, on different substrates, and to connect the emitter and the photodetector through conductors connecting the two substrates. For example, the two substrates could be fixed and electrically connected to a support, for example another substrate, and connected between themselves through the support. However, such a structure uses space. An advantage of the embodiments described in relation with Figures 1 and 2 to 6 is that it permits to form a device smaller and more compact than in the case of two different substrates.

Various embodiments and variants have been described. Those skilled in the art will understand that certain features of these embodiments can be combined and other variants will readily occur to those skilled in the art.

Finally, the practical implementation of the embodiments and variants described herein is within the capabilities of those skilled in the art based on the functional description provided hereinabove.

## Claims

1. An optoelectronic device (10, 50, 60, 70, 90) comprising, on a same substrate (18), a light emitter (12) and a photodetector (14, 92), at least one among the emitter and the photodetector being based on semiconductor nanoparticles.

2. A method of manufacturing an optoelectronic device comprising the formation, on a same substrate (18), of a light emitter (12) and of a photodetector (14, 92), at least one among the emitter and the photodetector being based on semiconductor nanoparticles.

3. The device according to claim 1 or the method according to claim 2, wherein each photodetector (14, 92) or emitter (12) based on semiconductor nanoparticles comprises a layer (26, 74) of semiconductor nanoparticles, a hole transport layer (30, 78), an electron transport layer (28, 76) and electrodes (32, 34, 80, 82).

4. The device according to claim 1 or 3 or the method according to claim 2 or 3, wherein the device comprises an emitter (12) based on semiconductor nanoparticles and a semiconductor-based photodetector (14).

5. The device or method according to claim 4, wherein the photodetector (14) comprises a photodiode.

6. The device or method according to claim 4 or 5, wherein the photodetector (14) does not comprise semiconductor nanoparticles.

7. The device or method according to any of claims 4 to 6, wherein the light emitter (12) and the photodetector (14, 92) are side by side on the substrate.

8. The device or method according to any of claims 4 to 6, wherein the light emitter (12) and the photodetector (14, 92) are stacked.

9. The device according to claim 1 or 3 or method according to claim 2 or 3, wherein both the light emitter (12) and the photodetector (14, 92) are based on semiconductor nanoparticles, and are stacked.

10. The device or method according to claim 9, wherein both the light emitter (12) and the photodetector (14, 92) are separated by an insulating layer (84).

11. The device according to any of claims 1 and 3 to 10 or method according to any of claims 2 to 10, wherein the substrate (91) is curved.

12. The device according to any of claims 1 and 3 to 11 or method according to any of claims 2 to 11, wherein the core of the semiconductor particles is made of a material among the following or an alloy of materials among the following: CdSe, CdS, CdTe, CdSeS, CdTeSe, AgS, ZnO, ZnS, ZnSe, CuInS, CuInSe, CuInGaS, CuInGaSe, PbS, PbSe, PbSeS, PbTe, InAsSb, InAs, InSb, InGaAs, InP, InGaP, InAlP, InGaAlP, InZnS, InZnSe, InZnSeS, HgTe, HgSe, HgSeTe, Ge, Si, and/or the shell of the semiconductor particles is made of a material among the following or an alloy of materials among the following: CdSe, CdS, CdTe, CdSeS, CdTeSe, AgS, ZnO, ZnS, ZnSe, CuInS, CuInSe, CuInGaS, CuInGaSe, PbS, PbSe, PbSeS, PbTe, InAsSb, InAs, InSb, InGaAs, InP, InGaP, InAlP, InGaAlP, InZnS, InZnSe, InZnSeS, HgTe, HgSe, HgSeTe, Ge, Si.

13. A spectrometer comprising a device according to any of claims 1, and 3 to 12.

14. An image sensor comprising a device according to any of claims 1, and 3 to 12.

15. A hyperspectral image sensor comprising a device according to any of claims 1, and 3 to 12.

16. A multispectral image sensor comprising a device according to any of claims 1, and 3 to 12.

17. A proximity sensor comprising a device according to any of claims 1, and 3 to 12.

18. A light communication device comprising a device according to any of claims 1, and 3 to 12.

19. A time of flight sensor comprising a device according to any of claims 1, and 3 to 12.

20. A finger print sensor comprising a device according to any of claims 1, and 3 to 12.

21. A face-ID sensor comprising a device according to any of claims 1, and 3 to 12.

22. A medical, health, or wellness sensor comprising a device according to any of claims 1, and 3 to 12.
